# EUROPEAN PATENT SPECIFICATION

(11) **EP 3 747 391 B1**
(45) Date of publication and mention of the grant of the patent: **24.12.2025**
(21) Application number: 20175905.7
(22) Date of filing: 26.10.2016
(51) Int. Cl.: A61B 90/50, A61B 90/57, A61B 90/25, A61B 18/00, A61B 18/18, A61B 18/14

(54) **APPARATUSES FOR SECURING A MEDICAL DEVICE AND RELATED METHODS THEREOF**
VORRICHTUNGEN ZUR SICHERUNG EINER MEDIZINISCHEN VORRICHTUNG UND ZUGEHÖRIGE VERFAHREN DAVON
APPAREILS POUR MAINTENIR UN DISPOSITIF MÉDICAL ET LEURS PROCÉDÉS ASSOCIÉS

(30) Priority: 26.10.2015 US 201562246355 P
(43) Date of publication of application: 09.12.2020
(62) Divisional of application: 16860698.6
(73) Proprietor: Neuwave Medical, Inc., Madison, WI 53704 (US)
(72) Inventor: BISSING, Jeff, Madison, WI 53704 (US); THOM, Mark, Madison, WI 53704 (US); SCHEFELKER, Richard W., Madison, WI 53704 (US); THIEL, Matthew, Madison, WI 53704 (US); SCHANING, Matt, Madison, WI 53704 (US); FERGUSON, J. Scott, Madison, WI 53704 (US)
(74) Representative: Murgitroyd & Company

(56) References cited:
- WO-A1-2012/159088
- US-A- 5 540 649
- US-A- 5 597 146
- US-A1- 2014 046 174

## Description

### FIELD OF THE INVENTION

The present invention relates to an apparatus for securing a medical device. In particular, the present invention relates to apparatuses for securing medical devices in a desired manner, wherein the apparatuses having an elongated main body, an anchoring region, a medical device attachment region, and a connection region. Such apparatuses are useful for securing a particular medical device (e.g., a microwave energy delivery device) in a favorable position while conducting a medical procedure with such a medical device (e.g., ablation of peripheral lung tissue).

### BACKGROUND OF THE INVENTION

Generally, medical procedures (e.g., ablation of peripheral lung tissue) involving the use of medical devices (e.g., energy delivery devices) require either a physician or a person assisting the physician to physically hold one or more of such devices in a desired favorable position for extended periods of time.

US 5,597,146 A describes a rail-mountable support assembly including a universal positioning arm for holding a surgical instrument such as an endoscope or retractor during a surgical procedure. Coarse adjustment of instrument X-Y position across the sterile field above an operating table is provided by an articulated arm which is rotatably coupled to an upright support shaft. Fine adjustment of surgical instrument elevation above the operating table is provided by a reversible drive motor which extends and retracts the instrument along the longitudinal axis of the support arm. The insertion orientation of the instrument is adjustable by a rotatable coupling. Release of the articulated arm for coarse positioning can be performed manually by actuation of an arm-mounted switch, and fine positioning can be performed manually by arm-mounted switches or by foot pressure applied to floor switches by the surgeon.

Apparatuses configured to permit the securing of such medical devices at a desired favorable location are needed.

### SUMMARY OF THE INVENTION

The present invention relates to an apparatus for securing a medical device. In particular, the present invention relates to apparatuses for securing medical devices in a desired manner, wherein the apparatuses having an elongated main body, an anchoring region, a medical device attachment region, and a connection region. Such apparatuses are useful for securing a particular medical device (e.g., a microwave energy delivery device) in a favorable position while conducting a medical procedure with such a medical device (e.g., ablation of peripheral lung tissue).

According to an aspect of the invention, there is provided an apparatus according to claim 1.

According to another aspect of the invention, there is provided a kit according to claim 7.

According to a further aspect of the invention, there is provided a method according to claim 14.

Further features according to embodiments of the invention are defined in the dependent claims.

In certain embodiments, the present invention provides apparatuses for securing a medical device, comprising an elongated main body having a main body proximal end and a main body distal end, wherein the length of the main body is greater than the width of the main body, wherein the length of the main body is expandable or retractable, an anchoring region engaged with the main body proximal end, wherein the anchoring region is configured to lock onto an anchorable region, wherein upon locking with an anchorable region the anchoring region and elongated main body are locked into a fixed position, a medical device attachment region having a medical device attachment region distal end and a medical device attachment region proximal end, wherein the medical device attachment region proximal end is elongated such that the length of the medical device attachment region proximal end is greater than the width of the medical device attachment region proximal end, wherein the length of the medical device attachment region proximal end is expandable or retractable, wherein the medical device attachment region distal end is configured to secure a medical device, and a connection region engaging the main body distal end and the medical device attachment region proximal end, wherein the connection region is adjustable such that the medical device attachment region can lockably project in any direction therefrom (i.e. a radial projection from 0° to 360° and rotational projection from 0° to 360°).

The main body is not limited to particular size dimensions. In some embodiments, the main body has a length less that is extendable to 2 meters and retractable to 0.2 meters. In some embodiments, the main body has a width that is between 0.005 meters and 0.3 meters.

The anchoring region is not limited to a particular manner of locking onto an anchorable region. In some embodiments, the anchoring region has an anchoring region compressible region and an anchoring region compressor region, wherein the anchoring region compressor region is configured to apply a range of compression to the anchoring region compressible region such that the size of the anchoring region compressible region decreases with increased amounts of compression and increases with decreased amounts of compression, wherein the amount of applied compression is lockable.

The medical device attachment region distal end is not limited to a particular manner of securing a medical device.

In some embodiments, the medical device attachment region distal end is configured with a torsion based securing mechanism, wherein the torsion based securing mechanism naturally rests in a closed manner (e.g., similar to an alligator clamp or clip). In some embodiments, the torsion based securing mechanism is configured to be opened for purposes of accommodating a medical device, wherein releasing the torsion based securing mechanism from an opened manner naturally results in torsion based closing of the torsion based securing mechanism.

In some embodiments, the medical device attachment region distal end has a medical device attachment region distal end compressible region and a medical device attachment region distal end compressor region, wherein the medical device attachment region distal end compressor region is configured to apply a range of compression to the medical device attachment region distal end compressible region such that the size of the medical device attachment region distal end compressible region decreases with increased amounts of compression and increases with decreased amounts of compression, wherein the amount of applied compression is lockable.

The medical device attachment region proximal end is not limited to particular size dimensions. In some embodiments, the medical device attachment region proximal end has a length less that is extendable to 2 meters and retractable to 0.5 meters. In some embodiments, the medical device attachment region proximal end has a width that is between 0.025 meters and 0.3 meters.

In some embodiments, the medical device attachment region further comprises one or more extensions for securing additional medical devices (e.g., additional energy delivery devices, light emitting objects, any other medical device). Such embodiments are not limited to a particular type or kind of extension for securing additional medical devices. In some embodiments, the extension is elongated with an additional device securing feature. In some embodiments, such an extension can be expanded or retracted to any desirable length.

In certain embodiments, the present invention provides methods for securing a medical device in a desire position, comprising securing such an apparatus to an anchorable region, securing a medical device with the medical device attachment region distal end, and adjusting the apparatus such that the secured medical device is in a desired position, wherein the adjusting comprises adjusting one or more of the following: adjusting the projection of the medical device attachment region from the connection region, adjusting the length of the medical device attachment region proximal end, and adjusting the length of the elongated main body. In some embodiments, the medical device is an energy delivery device configured to ablate tissue (e.g., a microwave energy delivery device or a radiofrequency energy delivery device).

In certain embodiments, the present disclosure provides methods (not claimed) for conducting a medical procedure with a medical device, comprising securing such apparatus to an anchorable region, securing a medical device with the medical device attachment region distal end, adjusting the apparatus such that the secured medical device is in a desired position, wherein the adjusting comprises adjusting one or more of the following: adjusting the projection of the medical device attachment region from the connection region, adjusting the length of the medical device attachment region proximal end, and adjusting the length of the elongated main body, and conducting the medical procedure with the medical device. In some embodiments, the medical device is an energy delivery device (e.g., a microwave energy delivery device or a radiofrequency energy delivery device). In some embodiments, the medical procedure is an ablation procedure, and the medical device is an energy delivery device.

In certain embodiments, the present invention provides kits comprising such an apparatus and a system, wherein the system comprises a primary catheter, wherein the primary catheter comprises a hollow primary lumen; a channel catheter, wherein the channel catheter is concentrically positioned within the hollow primary lumen, and wherein the channel catheter comprises a channel lumen; a steerable navigation catheter, wherein the steerable navigation catheter is configured to fit within the channel lumen, and wherein the steerable navigation catheter comprises a steerable tip and position sensing element; and an energy delivery device, wherein the energy delivery device is configured to fit within the channel lumen, and wherein the energy delivery device comprises, within the channel lumen, first, second and third conductors therein. In some embodiments, the energy delivery device is a microwave energy delivery device. In some embodiments, the energy delivery device is a radiofrequency energy delivery device. In some embodiments, the primary catheter comprises a bronchoscope. In some embodiments, the kit further comprises a processor configured to operate power delivery to the energy delivery device (e.g., microwave energy delivery device or radiofrequency energy delivery device).

In some embodiments, the energy delivery device (e.g., microwave energy delivery device or radiofrequency energy delivery device) is capable of delivering energy through the channel catheter.

In some embodiments, the energy delivery device is a microwave energy delivery device. In some embodiments, the microwave energy delivery device comprises a coolant channel and wherein the system comprises a coolant source in fluid communication with the coolant channel. In some embodiments, channel catheter comprises a braided material that provides flexibility. In some embodiments, the inner conductor is hollow. In some embodiments, the hollow of the inner conductor is in fluid communication with a coolant source. In some embodiments, the space between the inner and outer conductors comprises a dielectic material. In some embodiments, the space between the inner and outer conductors comprises air channels. In some embodiments, the kits further comprise a microwave power supply in electrical communication with the microwave energy delivery device.

In certain embodiments, the present disclosure provides methods (not claimed) for placing a microwave energy delivery device at a difficult to reach treatment site comprising providing such a kit, wherein the steerable navigation catheter is within the channel lumen, and the channel catheter is concentrically positioned within the hollow primary lumen; securing the apparatus to an anchorable region; securing the microwave energy delivery device with the medical device attachment region distal end; adjusting the apparatus such that the secured microwave energy delivery device is in a desired position, wherein the adjusting comprises adjusting one or more of the following: adjusting the projection of the medical device attachment region from the connection region, adjusting the length of the medical device attachment region proximal end, and adjusting the length of the elongated main body; inserting the primary catheter into an opening in a subject and directing the primary catheter towards the treatment site until further advance is constrained by the diameter of the primary catheter; advancing the channel catheter beyond the distal end of the primary catheter and extending the channel catheter to the treatment site; securing the distal end of the channel catheter at the treatment site; withdrawing the steerable navigation catheter through the channel lumen and out the proximal end of the channel catheter; and inserting the microwave energy delivery device through the channel lumen until the distal end of the microwave energy delivery device reaches the treatment site.

Such methods are not limited to a particular difficult to reach treatment site. In some embodiments, the difficult to reach treatment site comprises the periphery of the lung. In some embodiments, the difficult to reach treatment site comprises a peripheral lung nodule. In some embodiments, the lung nodule is accessed through the bronchial tree.

In certain embodiments, the present disclosure provides methods (not claimed) for treating a peripheral lung tissue region in a subject, comprising providing such a kit, wherein the steerable navigation catheter is within the channel lumen, and the channel catheter is concentrically positioned within the hollow primary lumen; securing the apparatus to an anchorable region; securing the energy delivery device with the medical device attachment region distal end; adjusting the apparatus such that the secured energy delivery device is in a desired position, wherein the adjusting comprises adjusting one or more of the following: adjusting the projection of the medical device attachment region from the connection region, adjusting the length of the medical device attachment region proximal end, and adjusting the length of the elongated main body; steering the energy delivery device through the subject's lung and positioning the microwave energy delivery device at a target peripheral lung tissue region, and ablating the target peripheral lung tissue region with energy from the microwave energy delivery device, wherein the steering is through the subject's mouth, through the subject's trachea, and through the subject's lung.

In some embodiments, the steering comprises advancing the hollow primary catheter having a hollow channel catheter therein through the subject's mouth, through the subject's trachea, and through the subject's lung until further advance is constrained by the diameter of the hollow primary catheter, wherein the hollow channel catheter has therein a steerable navigation catheter; advancing the hollow channel catheter having the steerable navigation catheter therein beyond the distal end of the hollow primary catheter and extending the hollow channel catheter having the steerable navigation catheter therein through the subject's lung and to the target peripheral lung tissue region; withdrawing the steerable navigation catheter from the hollow channel catheter; inserting the energy delivery device through the hollow channel catheter such that it is positioned at the target peripheral lung tissue region.

In some embodiments, advancing the hollow channel catheter having the steerable navigation catheter therein beyond the distal end of the hollow primary catheter and extending the hollow channel catheter having the steerable navigation catheter therein through the subject's lung comprises extending the hollow channel catheter having the steerable navigation catheter therein through one or more of primary bronchial tissue, secondary bronchial tissue, tertiary bronchial tissue, and bronchiole tissue. In some embodiments, the steerable navigation catheter controls the advancing.

In some embodiments, the microwave energy delivery device comprises a braided material. In some embodiments, microwave energy delivery device is flexible.

In some embodiments, ablating the target peripheral lung tissue region with energy from the microwave energy delivery device is controlled with a processor.

In some embodiments, the microwave energy delivery device is in electrical communication with an energy power supply. In some embodiments, the microwave energy delivery device comprises one or more coolant channels in fluid communication with a coolant source. In some embodiments, the microwave energy delivery device comprises an inner conductor and an outer conductor.

In some embodiments, the inner conductor is hollow. In some embodiments, the inner conductor is in fluid communication with a coolant source. In some embodiments, a dielectric material is positioned between the inner conductor and the outer conductor. In some embodiments, the inner conductor and the outer conductor comprise air channels.

In some embodiments, the target peripheral lung tissue region comprises lung nodule tissue. In some embodiments, the target peripheral lung tissue region comprises lung tumor tissue. In some embodiments, the target peripheral lung tissue region comprises lung lesion tissue. In some embodiments, the target peripheral lung tissue region comprises cancerous tissue.

In some embodiments, one or more stabilization and/or anchoring mechanisms are used to secure one or more of the hollow primary catheter, the hollow channel catheter, the steerable navigation catheter, and the energy delivery device at a desired tissue region.

In some embodiments, the desired tissue region is the target peripheral lung tissue region.

In some embodiments, the microwave energy delivery device is configured to detect an undesired rise in temperature within the energy delivery device and automatically or manually reduce such an undesired temperature rise through flowing of coolant through the one or more coolant channels. In some embodiments, the energy delivery device is a triaxial microwave probe. In some embodiments, the triaxial microwave probe comprises optimized tuning capabilities to reduce reflective heat loss. In some embodiments, the triaxial antenna comprises an inner conductor, a dielectric material, and an outer conductor, wherein the dielectric material is between the inner conductor and the outer conductor.

Additional embodiments are described herein.

### DESCRIPTION OF THE DRAWINGS

FIG. 1 shows a schematic presentation of the apparatus **1.** As shown, the apparatus **1** comprises an elongated main body **2,** an anchoring region **3,** a medical device attachment region **4,** and a connection region **5.**
FIG. 2 shows drawing of an apparatus **1,** having an elongated main body **2,** an anchoring region **3,** a medical device attachment region **4,** and a connection region **5.**
FIG. 3 shows a typical mounting procedure with an exemplary apparatus and the securing of a medical device with the apparatus.

### DETAILED DESCRIPTION OF THE INVENTION

The present invention relates to an apparatus for securing a medical device. In particular, the present invention relates to apparatuses for securing medical devices in a desired manner, wherein the apparatuses having an elongated main body, an anchoring region, a medical device attachment region, and a connection region. Such apparatuses are useful for securing a particular medical device (e.g., a microwave energy delivery device) in a desirable position while conducting a medical procedure with such a medical device (e.g., ablation of peripheral lung tissue).

The following discussion includes descriptions of the various embodiments of the apparatus in accordance with the principles of the present disclosure followed by a description of uses of the apparatus.

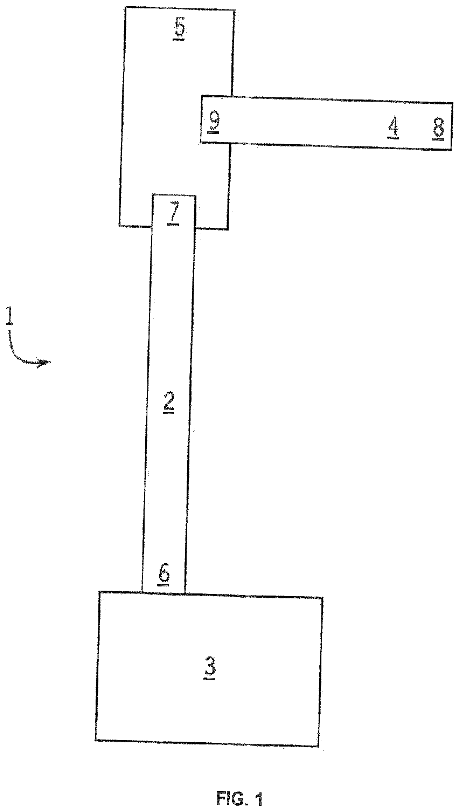

Referring to Figures 1-3, embodiments of the present invention provide apparatuses of the present invention.

Fig. 1 shows a schematic presentation of the apparatus **1.** As shown, the apparatus **1** comprises an elongated main body **2,** an anchoring region **3,** a medical device attachment region **4,** and a connection region **5.**

Still referring to Fig. 1, the elongated main body **2** has a main body proximal end **6** and a main body distal end **7.** The elongated main body **2** is not limited to a particular elongated size or configuration. In some embodiments, the elongated main body **2** has a length less that is extendable to approximately 2 meters (e.g., 1.5 meters, 1.6, 1.7, 1.75, 1.8, 1.9, 1.95, 1.99, 2.0, 2.01, 2.03, 2.5, 3, 5, 10, 15, etc.) and retractable to approximately 0.2 meters (e.g., 0.01 meters, 0.05, 0.1, 0.15, 0.2, 0.3, 0.35, 0.4, 0.48, 0.5, 0.51, 0.55, 0.6, 1, 1.5, etc.). In some embodiments, the elongated main body **2** has a width that is between approximately 0.005 meters and 0.3 meters (e.g., between 0.01 and 0.5 meters, 0.015 and 0.4, 0.02 and 0.3, etc.). The elongated main body **2** is not limited to a particular weight. In some embodiments, the weight of the elongated main body **2** is such that it does not compromise the integrity of any function or purpose of the apparatus **1.**

The elongated main body **2** is not limited to a particular manner for extending or retracting its length. In some embodiments, the elongated main body **2** utilizes, for example, a telescoping hydraulic system to expand or retract its length. In some embodiments, the elongated main body **2** is configured to lock its length at any desired distance.

The elongated main body **2** is not limited to a particular material composition. Indeed, those of skill in the art would readily know of an applicable type of material composition for the elongated main body **2.** In some embodiments, the elongated main body **2** is made of any suitable metal (e.g., stainless steel, titanium, niobium, tantalum, nitinol, copper, and/or a mixture thereof). In some embodiments, the elongated main body **2** is made of plastic. In some embodiments, the elongated main body **2** is made of a mixture of metal and plastic. In some embodiments, the material composition of the elongated main body **2** is such that it does not compromise the integrity of any function or purpose of the apparatus **1.**

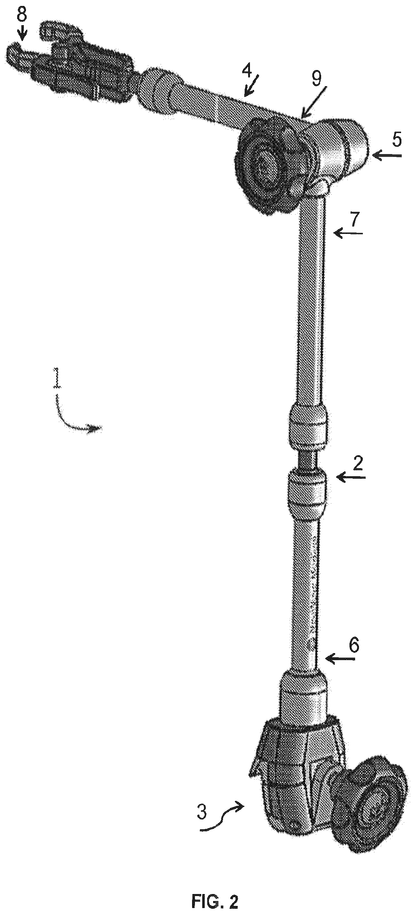

Fig. 2 shows drawing of an apparatus **1,** having an elongated main body **2,** an anchoring region **3,** a medical device attachment region **4,** and a connection region **5.** As can be seen, the elongated main body **2** has therein a main body proximal end **6** and a main body distal end 7. Furthermore as can be seen, the length of the elongated main body **2** is greater than its width. In addition, within this embodiment, the elongated main body **2** is divided at its mid-region thereby facilitating the expanding or retracting of its length (e.g., via a hydraulic telescoping design).

Referring again to Fig. 1, the anchoring region **3** is shown engaged with the main body proximal end **6.** The anchoring region 3 is not limited to particular manner of engaging with the main body proximal end **6.** In some embodiments, the engagement between the anchoring region **3** and the main body proximal end **6** is such that it does not compromise the integrity of any function or purpose of the apparatus **1.**

The anchoring region **3** is configured such that it permits the apparatus **1** as a whole to attach onto or with an anchorable region. The anchoring region **3** is not limited to attaching onto or with a particular type of anchorable region. Examples of anchorable regions for which the anchoring region **3** is able to attach onto or with include, but are not limited to, a medical procedure table or tray, a medical procedure device able to bear the weight of the apparatus **1,** a patient chair or bed, railing on a patient operating table, etc. In some embodiments, the anchorable region is any type of region for which the anchoring element **3** can attach onto or with such that it does not compromise the integrity of any function or purpose of the apparatus **1.**

In some embodiments, following attachment onto or with an anchorable region, the anchoring region **3** is able to lock such attachment such that absent unlocking the anchoring region 3 is immobilized. The anchoring region is not limited to a particular manner of locking the anchoring region 3 following attachment onto or with an anchorable region.

In some embodiments, the anchoring region attaches onto or with an anchorable region through a fixed / movable mechanism In some embodiments, the anchoring region attaches onto or with an anchorable region through a compression based mechanism. For example, in some embodiments, the anchoring region has an anchoring region compressible region and an anchoring region compressor region, wherein the anchoring region compressor region is configured to apply a range of compression to the anchoring region compressible region such that the size of the anchoring region compressible region decreases with increased amounts of compression and increases in size with decreased amounts of compression. Indeed, in some embodiments, the compression based mechanism resembles a clamping mechanism wherein tension is applied to the clamp thereby decreasing the size of the clamping region which thereby permits a tight adherence between the anchoring region and the anchorable region.

Still referring to Fig. 1, the anchoring region **3** is not limited to a particular size or configuration. In some embodiments, the size or configuration of the anchoring region **3** is such that it is able to attach onto or with an anchorable region region whereby such attachment is sufficient to immobilize the anchoring region **3.** In some embodiments, the size or configuration of the anchoring region **3** is such that it is able to immobilize the anchoring region **3** such that it does not compromise the integrity of any function or purpose of the apparatus **1.** In some embodiments, the width and height of the anchoring region **3** are independently between approximately 0.025 meters (e.g., between 0.01 and 0.5 meters, 0.015 and 0.4, 0.02 and 0.3, etc.) and approximately 2 meters (e.g., 1.5 meters, 1.6, 1.7, 1.75, 1.8, 1.9, 1.95, 1.99, 2.0, 2.01, 2.03, 2.5, 3, 5, 10, 15, etc.). The anchoring region **3** is not limited to a particular weight. In some embodiments, the weight of the anchoring region **3** is such that it does not compromise the integrity of any function or purpose of the apparatus **1.**

The anchoring region **3** is not limited to a particular material composition. Indeed, those of skill in the art would readily know of an applicable type of material composition for the anchoring region **3.** In some embodiments, the anchoring region **3** is made of any suitable metal (e.g., stainless steel, titanium, niobium, tantalum, nitinol, copper, and/or a mixture thereof). In some embodiments, the anchoring region **3** is made of plastic. In some embodiments, the anchoring region **3** is made of a mixture of metal and plastic. In some embodiments, the material composition of the anchoring region **3** is such that it does not compromise the integrity of any function or purpose of the apparatus **1.**

Referring to Fig. 2, the anchoring element **3** is shown engaged with the main body proximal end **6.** As can be seen, within this embodiment, the anchoring element **3** has thereon a fastening knob which can be used to increase compression within a compressible region thereby facilitating attachment of the anchoring region **3** onto or with an anchorable region.

Referring again to Fig. 1, a medical device attachment region **4** having a medical device attachment region distal end **8** and a medical device attachment region proximal end **9** is shown. In addition, as can be seen, the medical device attachment region proximal end **9** is shown engaged with the connection region **5.**

The medical device attachment region proximal end **9** is not limited to a particular elongated size or configuration. In some embodiments, as can be seen from Fig. 1, the medical device attachment region proximal end **9** is elongated. In some embodiments, the medical device attachment region proximal end **9** has a length less that is extendable to approximately 2 meters (e.g., 1.5 meters, 1.6, 1.7, 1.75, 1.8, 1.9, 1.95, 1.99, 2.0, 2.01, 2.03, 2.5, 3, 5, 10, 15, etc.) and retractable to approximately 0.5 meters (e.g., 0.1 meters, 0.2, 0.3, 0.35, 0.4, 0.48, 0.5, 0.51, 0.55, 0.6, 1, 1.5, etc.). In some embodiments, the medical device attachment region proximal end **9** has a width that is between approximately 0.025 meters and 0.3 meters (e.g., between 0.01 and 0.5 meters, 0.015 and 0.4, 0.02 and 0.3, etc.). The medical device attachment region proximal end **9** is not limited to a particular weight. In some embodiments, the weight of the medical device attachment region proximal end **9** is such that it does not compromise the integrity of any function or purpose of the apparatus **1.**

The medical device attachment region proximal end **9** is not limited to a particular manner for extending or retracting its length. In some embodiments, the medical device attachment region proximal end **9** utilizes, for example, a telescoping hydraulic system to expand or retract its length. In some embodiments, the medical device attachment region proximal end **9** is configured to lock its length at any desired distance. In some embodiments, the medical device attachment region proximal end **9** is flexible such that it can be bent or shaped in any desired manner (e.g., a goose-neck formation).

The medical device attachment region proximal end **9** is not limited to a particular material composition. Indeed, those of skill in the art would readily know of an applicable type of material composition for the medical device attachment region proximal end 9. In some embodiments, the medical device attachment region proximal end **9** is made of any suitable metal (e.g., stainless steel, titanium, niobium, tantalum, nitinol, copper, and/or a mixture thereof). In some embodiments, the medical device attachment region proximal end **9** is made of plastic. In some embodiments, the medical device attachment region proximal end **9** is made of a mixture of metal and plastic. In some embodiments, the medical device attachment region proximal end **9** is made of a flexible material which permits the region to be shaped in any desired manner. In some embodiments, the material composition of the medical device attachment region proximal end **9** is such that it does not compromise the integrity of any function or purpose of the apparatus **1.**

Referring to Fig. 2, the medical device attachment region proximal end **9** is shown engaged with the connection region **5** and the medical device attachment region distal end **9.** Furthermore as can be seen, the length of the medical device attachment region proximal end **9** is greater than its width.

Referring again to Fig. 1, the medical device attachment region distal end **8** is shown engaged with the medical device attachment region proximal end **9.** The medical device attachment region distal end **8** is not limited to particular manner of engaging with the medical device attachment region proximal end **9.** In some embodiments, the engagement between the medical device attachment region distal end **8** and the medical device attachment region proximal end **9** is such that it does not compromise the integrity of any function or purpose of the apparatus **1.**

The medical device attachment region distal end **8** is configured such that it permits a medical device to secure with the medical device attachment region distal end **8** and, as such, the apparatus **1** as a whole. The medical device attachment region distal end **8** is not limited to securing a particular type of medical device (see below for more detail).

In some embodiments, following securing of a medical device with the medical device attachment region distal end **8,** the medical device attachment region distal end **8** is able to lock such securing such that absent unlocking the medical device is immobilized with the medical device attachment region distal end **8.** The medical device attachment region distal end 8 is not limited to a particular manner of locking a medical device following securing with the medical device attachment region distal end **8.**

In some embodiments, the medical device attachment region distal end utilizes a torsion based mechanism for securing and locking a medical device. For example, in some embodiments, the medical device attachment region distal end has a mechanism that naturally rests in a locked position. In such embodiments, the torsion based mechanism is configured to be opened (e.g., manual opening) to a certain dimension such that it can accommodate a medical device (e.g., for purposes of securing the medical device within the medical device attachment region distal end). In such embodiments, following opening of the torsion based mechanism a release of such opening results in a closing of the mechanism (e.g., torsion based closing). In some embodiments, following positioning of a medical device within the medical device attachment region distal end such, closing of the mechanism results in a torsion based securing. In some embodiments, the torsion based mechanism a spring based. In some embodiments, the torsion based mechanism resembles an alligator clamp or clip.

In some embodiments, the medical device attachment region distal end utilizes a magnet based mechanism for securing and locking a medical device.

In some embodiments, the medical device attachment region distal end utilizes a compression based mechanism for securing and locking a medical device. For example, in some embodiments, the medical device attachment region distal end has a medical device attachment region distal end compressible region and a medical device attachment region distal end compressor region, wherein the medical device attachment region distal end compressor region is configured to apply a range of compression to the medical device attachment region distal end compressible region such that the size of the medical device attachment region distal end compressible region decreases with increased amounts of compression and increases in size with decreased amounts of compression. Indeed, in some embodiments, the compression based mechanism resembles a clamping mechanism wherein tension is applied to the clamp thereby decreasing the size of the clamping region which thereby permits a tight securing of a medical device within the medical device attachment region distal end.

In some embodiments, the mechanism within the medical device attachment region distal end for securing and locking a medical device is configured such that it does not obscure and/or hinder access to a secured medical device. For example, in some embodiments, the mechanism within the medical device attachment region distal end for securing and locking a medical device is sized such that a user can manipulate and/or read a medical device that is secured therein. In some embodiments, the mechanism within the medical device attachment region distal end for securing and locking a medical device is no larger than one or two adult human fingers.

Still referring to Fig. 1, the medical device attachment region distal end **8** is not limited to a particular size or configuration. In some embodiments, the size or configuration of the medical device attachment region distal end **8** is such that it is able to secure any type of medical device having any kind of size. In some embodiments, the size or configuration of the medical device attachment region distal end **8** is such that it is able to secure a medical device without compromising the integrity of any function or purpose of the apparatus **1.** In some embodiments, the width and height of the medical device attachment region distal end **8** are independently between approximately 0.025 meters (e.g., between 0.01 and 0.5 meters, 0.015 and 0.4, 0.02 and 0.3, etc.) and approximately 2 meters (e.g., 1.5 meters, 1.6, 1.7, 1.75, 1.8, 1.9, 1.95, 1.99, 2.0, 2.01, 2.03, 2.5, 3, 5, 10, 15, etc.). The medical device attachment region distal end **8** is not limited to a particular weight. In some embodiments, the weight of the medical device attachment region distal end **8** is such that it does not compromise the integrity of any function or purpose of the apparatus **1.**

The medical device attachment region distal end **8** is not limited to a particular material composition. Indeed, those of skill in the art would readily know of an applicable type of material composition for the medical device attachment region distal end **8.** In some embodiments, the medical device attachment region distal end **8** is made of any suitable metal (e.g., stainless steel, titanium, niobium, tantalum, nitinol, copper, and/or a mixture thereof). In some embodiments, the medical device attachment region distal end **8** is made of plastic. In some embodiments, the medical device attachment region distal end **8** is made of a mixture of metal and plastic. In some embodiments, the material composition of the medical device attachment region distal end **8** is such that it does not compromise the integrity of any function or purpose of the apparatus **1.**

Referring to Fig. 2, the medical device attachment region distal end **8** is shown engaged with the medical device attachment region proximal end **9.** As can be seen, within this embodiment, the medical device attachment region distal end **8** has thereon a dual prong that is able to be increased or decreased in size for purposes of applying or relieving a compression force onto a medical device (e.g., for purposes of securing or releasing a secured medical device with or from the medical device attachment region distal end **8**).

In some embodiments, the medical device attachment region further comprises one or more extensions for securing additional medical devices (e.g., additional energy delivery devices, light emitting objects, any other medical device). Such embodiments are not limited to a particular type or kind of extension for securing additional medical devices. In some embodiments, the extension is elongated with an additional device securing feature. In some embodiments, such an extension can be expanded or retracted to any desirable length. In some embodiments, the one or more extensions attach with the medical device attachment region via a bolting mechanism.

Referring again to Fig. 1, the connection region **3** is shown engaged with the medical device attachment region distal end **9** and the main body distal end **7.** The connection region **3** is not limited to particular manner of engaging with the medical device attachment region distal end **9** and the main body distal end **7.** In some embodiments, the engagement between the connection region **3** and the medical device attachment region distal end **9** and the main body distal end **7** is such that it does not compromise the integrity of any function or purpose of the apparatus **1.**

In some embodiments, the engagement between the connection region **3** and main body distal end **7** is such that it immobilizes the elongated main body **2** in a fixed position.

The engagement between the connection region **3** and the medical device attachment region distal end **9** is such that it renders the medical device attachment region **4** capable of projection therefrom in any desired manner. For example, the engagement between the connection region **3** and the medical device attachment region distal end **9** is such that the medical device attachment region **4** can be positioned in any projection therefrom (i. e. a radial projection from 0° to 360°). The engagement between the connection region **3** and the medical device attachment region distal end **9** is also such that the medical device attachment region **4** can be rotated about the axis of the connection region **3** (i.e. a rotational projection from 0° to 360°). Indeed, in some embodiments, the engagement between the connection region **3** and the medical device attachment region distal end **9** is such that the medical device attachment region **4** can be articulated in any desired manner.

In some embodiments, the connection region **3** is configured to lock the medical device attachment region **4** in a fixed projection. The connection region **3** is not limited to a particular manner of locking the medical device attachment region **4** in a fixed projection. In some embodiments, the connection region **3** has utilizes a tension applicator based mechanism for locking and unlocking the medical device attachment region **4** in a fixed projection.

Still referring to Fig. 1, the connection region **3** is not limited to a particular size or configuration. In some embodiments, the size or configuration of the connection region **3** is such that it is able to engage the medical device attachment region distal end **9** and the main body distal end **7** without compromising the integrity of any function or purpose of the apparatus **1.** In some embodiments, the size or configuration of the connection region **3** is such that it is able to engage the main body distal end **7** such that it immobilizes the elongated main body **2** in a fixed position. In some embodiments, the size or configuration of the connection region **3** is such that it renders the medical device attachment region **4** capable of projection therefrom in any desired manner.

In some embodiments, the width and height of the connection region **3** are independently between approximately 0.025 meters (e.g., between 0.01 and 0.5 meters, 0.015 and 0.4, 0.02 and 0.3, etc.) and approximately 2 meters (e.g., 1.5 meters, 1.6, 1.7, 1.75, 1.8, 1.9, 1.95, 1.99, 2.0, 2.01, 2.03, 2.5, 3, 5, 10, 15, etc.). The connection region **3** is not limited to a particular weight. In some embodiments, the weight of the connection region **3** is such that it does not compromise the integrity of any function or purpose of the apparatus **1.**

The connection region **3** is not limited to a particular material composition. Indeed, those of skill in the art would readily know of an applicable type of material composition for the connection region **3.** In some embodiments, the connection region **3** is made of any suitable metal (e.g., stainless steel, titanium, niobium, tantalum, nitinol, copper, and/or a mixture thereof). In some embodiments, the connection region **3** is made of plastic. In some embodiments, the connection region **3** is made of a mixture of metal and plastic. In some embodiments, the material composition of the connection region **3** is such that it does not compromise the integrity of any function or purpose of the apparatus **1.**

Referring to Fig. 2, the connection region 3 is shown engaged with the medical device attachment region distal end 9 and the main body distal end 7. As can be seen, the engagement between the connection region 3 and the main body distal end 7 is such that it immobilizes the elongated main body 2 in a fixed position. As can be seen, the engagement between the connection region 3 and the medical device attachment region distal end 9 is such that the medical device attachment region 4 is projecting therefrom at approximately a 90° angle. Furthermore, as can be seen, the connection region 3 has thereon a fastening knob which can be used to increase or decrease tension onto the connection region 3 thereby locking or unlocking the medical device attachment region 4 in or from a desired projection.

The apparatuses of the present invention are not limited to securing a particular type of medical device. Indeed, the apparatuses of the present invention can be configured to secure any type or size of medical device.

In some embodiments, the medical device is an energy delivery device. The present invention is not limited to securing a particular type or kind of energy delivery device. Indeed, such embodiments contemplate the use of any type of device configured to deliver (e.g., emit) energy (e.g., ablation device, surgical device, etc.) (see, e.g., U.S. Patent Nos. 7,101,369 , 7,033,352, 6,893,436 , 6,878,147 , 6,823,218 , 6,817,999 , 6,635,055 , 6,471,696 , 6,383,182 , 6,312,427 , 6,287,302 , 6,277,113 , 6,251,128 , 6,245,062 , 6,026,331 , 6,016,811 , 5,810,803 , 5,800,494 , 5,788,692 , 5,405,346 , 4,494,539 , U.S. Patent Application Serial Nos. 11/728,460 , 11/728,457 , 11/728,428 , 11/237,136, 11/236,985 , 10/980,699 , 10/961,994 , 10/961,761 , 10/834,802, 10/370,179 , 09/847,181 ; Great Britain Patent Application Nos. 2,406,521 , 2,388,039 ; European Patent No. 1395190 ; and International Patent Application Nos. WO 06/008481 , WO 06/002943 , WO 05/034783 , WO 04/112628 , WO 04/033039 , WO 04/026122 , WO 03/088858 , WO 03/039385 WO 95/04385). Such devices include any and all medical, veterinary, and research applications devices configured for energy emission, as well as devices used in agricultural settings, manufacturing settings, mechanical settings, or any other application where energy is to be delivered.

In some embodiments, the apparatuses of the present invention are configured to secure energy delivery devices having therein antennae configured to emit energy (e.g., microwave energy, radiofrequency energy, radiation energy). The apparatuses are not limited to secure energy delivery devices having particular types or designs of antennae (e.g., ablation device, surgical device, etc.). In some embodiments, the apparatuses are configured to secure energy delivery devices having linearly shaped antennae (see, e.g., U.S. Patent Nos. 6,878,147 , 4,494,539 , U.S. Patent Application Serial Nos. 11/728,460 , 11/728,457 , 11/728,428 , 10/961,994 , 10/961,761 ; and International Patent Application No., WO 03/039385). In some embodiments, the apparatuses are configured to secure energy delivery devices having non-linearly shaped antennae (see, e.g., U.S. Patent Nos. 6,251,128 , 6,016,811 , and 5,800,494 , U.S. Patent Application Serial No. 09/847,181 , and International Patent Application No. WO 03/088858). In some embodiments, the apparatuses are configured to secure energy delivery devices having antennae with horn reflection components (see, e.g., U.S. Patent Nos. 6,527,768 , 6,287,302). In some embodiments, the apparatuses are configured to secure energy delivery devices having antennae with a directional reflection shield (see, e.g., U.S. Patent No. 6,312,427).

In some embodiments, the apparatuses are configured to secure energy delivery devices comprise coaxial transmission lines. Such devices are not limited to particular configurations of coaxial transmission lines. Examples of coaxial transmission lines include, but are not limited to, coaxial transmission lines developed by Pasternack, Micro-coax, and SRC Cables. In some embodiments, the coaxial transmission line has a center conductor, a dielectric element, and an outer conductor (e.g., outer shield). In some embodiments, the energy delivery devices comprise flexible coaxial transmission lines (e.g., for purposes of positioning around, for example, pulmonary veins or through tubular structures) (see, e.g., U.S. Patent Nos. 7,033,352 , 6,893,436 , 6,817,999 , 6,251,128 , 5,810,803 , 5,800,494). In some embodiments, the energy delivery devices utilize antennae having rigid coaxial transmission lines (see, e.g., U.S. Patent No. 6,878,147 , U.S. Patent Application Serial Nos. 10/961,994 , 10/961,761 , and International Patent Application No. WO 03/039385).

In some embodiments, the energy delivery devices have a coaxial transmission line positioned within the antenna, and a coaxial transmission line connecting with the antenna. In some embodiments, the size of the coaxial transmission line within the antenna is larger than the coaxial transmission line connected with the antenna. The coaxial transmission line within the antenna and the coaxial transmission line connecting with the antenna are not limited to particular sizes. For example, in some embodiments, whereas the coaxial transmission line connected with the antenna is approximately 0.81 mm (0.032 inches), the size of the coaxial transmission line within the antenna is larger than 0.81mm (0.032 inches) (e.g., 1.27 mm (0.05 inches), 1.9mm (0.075 inches), 2.5mm (0.1 inches), 12.7mm (0.5 inches)). In some embodiments, the coaxial transmission line within the antenna has an inner conductor that is stiff and thick. In some embodiments, the end of the coaxial transmission line within the antenna is sharpened for percutaneous use. In some embodiments, the dielectric coating of the coaxial transmission line within the antenna is PTFE (e.g., for purposes of smoothing transitions from a cannula to an inner conductor (e.g., a thin and sharp inner conductor)). In some embodiments, the shape of the coaxial transmission line and/or the dielectric element is selected and/or adjustable to fit a particular need.

In some embodiments, the energy delivery devices have a triaxial transmission line. In some embodiments, the apparatuses are configured to secure energy delivery devices having a triaxial microwave probe design where the outer conductor allows improved tuning of the antenna to reduce reflected energy through the transmission line. This improved tuning reduces heating of the transmission line allowing more power to be applied to the tissue and/or a smaller transmission line (e.g. narrower) to be used. Further, the outer conductor may slide with respect to the inner conductors to permit adjustment of the tuning to correct for effects of the tissue on the tuning. In some embodiments, and outer conductor is stationary with respect to the inner conductors. In some embodiments, the apparatuses are configured to secure energy delivery devices having antennae with a probe having a first conductor and a tubular second conductor coaxially around the first conductor but insulated therefrom (e.g. insulated by a dielectric material and/or coolant). A tubular third conductor is fit coaxially around the first and second conductors. The first conductor may extend beyond the second conductor into tissue when a proximal end of the probe is inserted into a body. The second conductor may extend beyond the third conductor into the tissue to provide improved tuning of the probe limiting power dissipated in the probe outside of the exposed portions of the first and second conductors. The third tubular conductor may be a channel catheter for insertion into the body or may be separate from a channel catheter. In some embodiments, the apparatuses are configured to secure energy delivery devices comprising first, second, and third conductors sufficiently flexible to navigate a winding path (e.g. through a branched structure within a subject (e.g. through the brachial tree)). In some embodiments, the first and second conductors may fit slidably within the third conductor. In some embodiments, the apparatuses are configured to secure energy delivery devices having a probe that facilitates tuning of the probe in tissue by sliding the first and second conductors inside of the third conductor. In some embodiments, the probe includes a lock attached to the third conductor to adjustably lock a sliding location of the first and second conductors with respect to the third conductor. In some embodiments, the apparatuses are configured to secure energy delivery devices having a triaxial transmission line, as described in U.S. Pat. No. 7,101,369, U.S. Pat. App. No. 2007/0016180, U.S. Pat. App. No.2008/0033424, U.S. Pat. App. No. 20100045558 , U.S. Pat. App. No. 20100045559.

In certain embodiments, the present invention provides methods for securing a medical device in a desire position, comprising securing an apparatus of the invention to an anchorable region, securing a medical device with the medical device attachment region distal end, and adjusting the apparatus such that the secured medical device is in a desired position, wherein the adjusting comprises adjusting one or more of the following: adjusting the projection of the medical device attachment region from the connection region, adjusting the length of the medical device attachment region proximal end, and adjusting the length of the elongated main body.

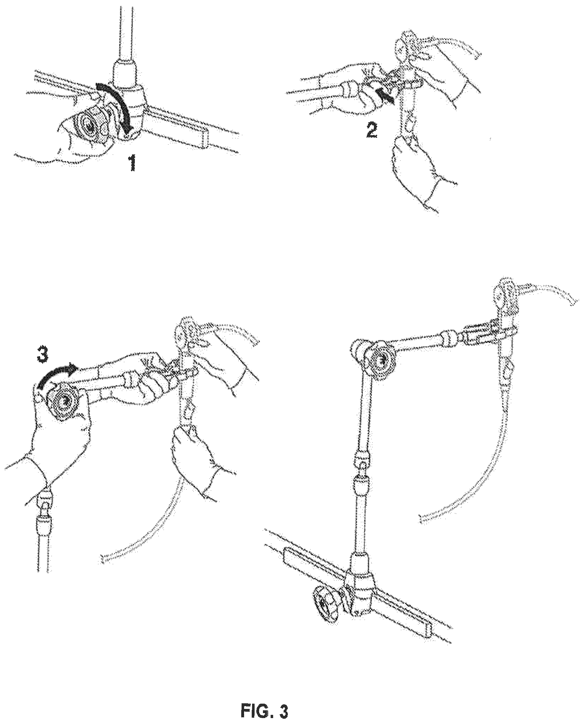

Indeed, Fig. 3 shows a typical mounting procedure with an apparatus of the present invention and the securing of a medical device with the apparatus. As shown in "1", the anchoring region of the apparatus is attached with an anchorable region. As shown, the anchoring region is positioned such that upon rotation of the rotational element a compressional force is applied thereby resulting in attachment of the apparatus onto the anchorable region. Next, as shown in "2", a medical device is secured within the medical device attachment region. Next, as shown in "3", the projection of the medical device attachment region from connection region is adjusted to a desired projection and locked into place.

The apparatuses of the present invention can be used, as such, within any type of medical procedure involving the use of a medical device. Indeed, such apparatuses greatly improve the efficiency of such medical procedures through fixing the medical device into a desired position and thereby removing the necessity of physically holding such a medical device at a desired location (e.g., by a physician, nurse, assistant, etc.).

In some embodiments, the apparatuses of the present invention are configured for use with systems comprising, for example, a primary catheter, wherein the primary catheter comprises a hollow primary lumen; a channel catheter, wherein the channel catheter is concentrically positioned within the hollow primary lumen, and wherein the channel catheter comprises a channel lumen; a steerable navigation catheter, wherein the steerable navigation catheter is configured to fit within the channel lumen, and wherein the steerable navigation catheter comprises a steerable tip and position sensing element; and a microwave energy delivery device, wherein the energy delivery device is configured to fit within the channel lumen, and wherein the microwave energy delivery device comprises, within the channel lumen, first, second and third conductors therein.

In some embodiments, any suitable endoscope or bronchoscope known to those in the art finds use as a primary catheter of such a system. In some embodiments, a primary catheter adopts characteristics of one or more endoscopes and/or bronchoscopes known in the art, as well as characteristics described herein. One type of conventional flexible bronchoscope is described in U.S. Pat. No. 4,880,015. The bronchoscope measures 790 mm in length and has two main parts, a working head and an insertion tube. The working head contains an eyepiece; an ocular lens with a diopter adjusting ring; attachments for suction tubing, a suction valve, and light source; and an access port or biopsy inlet, through which various devices and fluids can be passed into the working channel and out the distal end of the bronchoscope. The working head is attached to the insertion tube, which typically measures 580 mm in length and 6.3 mm in diameter. The insertion tube contains fiberoptic bundles, which terminate in the objective lens at the distal tip, light guides, and a working channel. Other endoscopes and bronchoscopes which may find use in such systems, or portions of which may find use with the present invention, are described in U.S. Pat. No. 7,473,219; U.S. Pat. No. 6,086,529; U.S. Pat. No. 4,586,491; U.S. Pat. No. 7,263,997; U.S. Pat. No. 7,233,820 ; and U.S. Pat. No. 6,174,307.

In some embodiments, the systems provide a channel catheter (a.k.a. guide catheter, sheath, sheath catheter, etc.). In some embodiments, a guide catheter is configured to fit within the lumen of a primary catheter and contains a channel lumen of sufficient diameter (e.g. 1 mm... 2 mm... 3 mm... 4 mm... 5 mm) to accommodate a steerable navigation catheter and/or one or more suitable tools (e.g. energy delivery device). In some embodiments, a channel catheter is of sufficient length to extend from an insertion site (e.g. mouth, incision into body of subject, etc.) through the trachea and/or bronchial tree to a treatment site in the peripheral lung (e.g. 50 cm... 75 cm... 1 m... 1.5 m... 2m). In some embodiments, a channel catheter is of sufficient length to extend beyond the reach of a primary catheter to reach a treatment site (e.g. peripheral lung tissue). In some embodiments, a channel catheter is highly flexible to access a circuitous route through a subject (e.g. through a branched structure, through the bronchial tree, etc.). In some embodiments, a channel catheter is constructed of braided material to provide both strength and flexibility, as is understood in the art. In some embodiments, a channel catheter comprises the outer conductor of a triaxial transmission line. In some embodiments, a channel catheter comprises a navigation and/or steering mechanism. In some embodiments, a channel catheter is without an independent means of navigation, position recognition, or maneuvering. In some embodiments, a channel catheter relies upon the primary catheter or steerable navigation catheter for placement.

In some embodiments, the systems provide a steerable navigation catheter. In some embodiments, a steerable navigation catheter is configured to fit within the lumen of a channel catheter. In some embodiments, a steerable navigation catheter has a similar diameter to energy transmission lines described herein (e.g. 0.2 mm... 0.5 mm... 1.0 mm... 1.5 mm... 2.0mm). In some embodiments, a steerable navigation catheter is of sufficient length to extend from an insertion site (e.g. mouth, incision into body of subject, etc.) to a treatment site (e.g. through the trachea and/or bronchial tree to a treatment site in the peripheral lung (e.g. 50 cm... 75 cm... 1 m... 1.5 m...2m). In some embodiments, a channel catheter is of sufficient length to extend beyond the reach of a primary catheter to reach a treatment site (e.g. peripheral lung tissue). In some embodiments, a steerable navigation catheter engages a channel catheter such that movement of the steerable navigation catheter results in synchronous movement of the channel catheter. In some embodiments, as a steerable navigation catheter is inserted along a path in a subject, the channel catheter surrounding the steerable navigation catheter moves with it. In some embodiments, a channel catheter is placed within a subject by a steerable navigation catheter. In some embodiments, a steerable navigation catheter can be disengaged from a channel catheter. In some embodiments, disengagement of a steerable navigation catheter and channel catheter allows movement of the steerable navigation catheter further along a pathway without movement of the channel catheter. In some embodiments, disengagement of a steerable navigation catheter and channel catheter allows retraction of the steerable navigation catheter through the channel catheter without movement of the channel catheter.

In some embodiments, all inserted components of a system or device are configured for movement along a narrow and circuitous path through a subject (e.g. through a branched structure, through the bronchial tree, etc.). In some embodiment, components comprise a flexible material configured for tight turning radiuses. In some embodiment, necessarily rigid components are reduced in size (e.g. short length) to allow for tight turning radiuses.

As such, in certain embodiments, the present disclosure provides methods (not claimed) for placing a microwave energy delivery device at a difficult to reach treatment site comprising providing an apparatus of the present invention and the above described system, wherein the steerable navigation catheter is within the channel lumen, and the channel catheter is concentrically positioned within the hollow primary lumen. In some embodiments, the methods involve securing the apparatus to an anchorable region; securing the microwave energy delivery device with the medical device attachment region distal end; adjusting the apparatus such that the secured microwave energy delivery device is in a desired position, wherein the adjusting comprises adjusting one or more of the following: adjusting the projection of the medical device attachment region from the connection region, adjusting the length of the medical device attachment region proximal end, and adjusting the length of the elongated main body; inserting the primary catheter into an opening in a subject and directing the primary catheter towards the treatment site until further advance is constrained by the diameter of the primary catheter; advancing the channel catheter beyond the distal end of the primary catheter and extending the channel catheter to the treatment site; securing the distal end of the channel catheter at the treatment site; withdrawing the steerable navigation catheter through the channel lumen and out the proximal end of the channel catheter; inserting the microwave energy delivery device through the channel lumen until the distal end of the microwave energy delivery device reaches the treatment site.

In some embodiments, the difficult to reach treatment site comprises the periphery of the lung and/or a peripheral lung nodule. In some embodiments, the lung nodule is accessed through the bronchial tree.

In certain embodiments, the present disclosure provides methods (not claimed) for treating a peripheral lung tissue region comprising providing an apparatus of the present invention and a system as described above, wherein the steerable navigation catheter is within the channel lumen, and the channel catheter is concentrically positioned within the hollow primary lumen. In some embodiments, the methods involve securing the apparatus to an anchorable region; securing the energy delivery device with the medical device attachment region distal end; adjusting the apparatus such that the secured energy delivery device is in a desired position, wherein the adjusting comprises adjusting one or more of the following: adjusting the projection of the medical device attachment region from the connection region, adjusting the length of the medical device attachment region proximal end, and adjusting the length of the elongated main body; steering the energy delivery device through the subject's lung and positioning the microwave energy delivery device at a target peripheral lung tissue region, and ablating the target peripheral lung tissue region with energy from the microwave energy delivery device, wherein the steering is through the subject's mouth, through the subject's trachea, and through the subject's lung.

In certain embodiments, the present invention provides kits comprising an apparatus of the present invention and a system comprises one or more of a primary catheter, wherein the primary catheter comprises a hollow primary lumen; a channel catheter, wherein the channel catheter is concentrically positioned within the hollow primary lumen, and wherein the channel catheter comprises a channel lumen; a steerable navigation catheter, wherein the steerable navigation catheter is configured to fit within the channel lumen, and wherein the steerable navigation catheter comprises a steerable tip and position sensing element; and a microwave energy delivery device, wherein the energy delivery device is configured to fit within the channel lumen, and wherein the microwave energy delivery device comprises, within the channel lumen, first, second and third conductors therein. **In** some embodiments, the primary catheter comprises a bronchoscope. **In** some embodiments, the channel catheter comprises a braided material that provides flexibility. **In** some embodiments, the inner conductor is hollow. **In** some embodiments, the hollow of the inner conductor is in fluid communication with a coolant source. **In** some embodiments, the space between the inner and outer conductors comprises a dielectric material. **In** some embodiments, the space between the inner and outer conductors comprises air channels.

**In** some embodiments, the systems further comprise a handle for manipulation of one or more of the primary catheter, the channel catheter, the steerable navigation catheter, and the microwave energy delivery device.

**In** some embodiments, the systems further comprise a processor configured to operate power delivery to the microwave energy delivery device.

**In** some embodiments, the systems further comprise a microwave power supply in electrical communication with the microwave energy delivery device.

## Claims

1. An apparatus (1) for securing a medical device, comprising
an elongated main body (2) having a main body proximal end (6) and a main body distal end (7), wherein the length of the main body is greater than the width of the main body,
an anchoring region (3) engaged with the main body proximal end, wherein the anchoring region is configured to lock onto an anchorable region, wherein upon locking with an anchorable region the anchoring region and elongated main body are locked into a fixed position,
a medical device attachment region (4) having a medical device attachment region distal end (8) and a medical device attachment region proximal end (9),
wherein the medical device attachment region proximal end is elongated such that the length of the medical device attachment region proximal end is greater than the width of the medical device attachment region proximal end,
wherein the medical device attachment region distal end is configured to secure a medical device, and
a connection region (5) engaging the main body distal end and the medical device attachment region proximal end, wherein the connection region is adjustable such that the medical device attachment region can lockably project in any direction therefrom, i.e. with a radial projection from 0° to 360° and a rotational projection from 0° to 360° about an axis of the connection region.

2. The apparatus of claim 1, wherein the main body has a width that is between 0.005 meters and 0.3 meters.

3. The apparatus of claim 1 or claim 2, wherein the anchoring region has an anchoring region compressible region and an anchoring region compressor region, wherein the anchoring region compressor region is configured to apply a range of compression to the anchoring region compressible region such that the size of the anchoring region compressible region decreases with increased amounts of compression and increases with decreased amounts of compression, wherein the amount of applied compression is lockable.

4. The apparatus of any preceding claim, wherein the medical device attachment region distal end is configured with a torsion based securing mechanism, wherein the torsion based securing mechanism naturally rests in a closed manner, wherein the torsion based securing mechanism is configured to be opened for purposes of accommodating a medical device, wherein releasing the torsion based securing mechanism from an opened manner naturally results in torsion based closing of the torsion based securing mechanism.

5. The apparatus of any preceding claim, wherein the medical device attachment region has thereon one or more extensions projecting therefrom, wherein the one or more extensions are configured to secure one or more medical devices.

6. The apparatus of any preceding claim, wherein the medical device attachment region proximal end has a width that is between 0.025 meters and 0.3 meters.

7. A kit comprising:
an apparatus (1) of any preceding claim; and
a system, wherein the system comprises:
a primary catheter, wherein the primary catheter comprises a hollow primary lumen;
a channel catheter, wherein the channel catheter is concentrically positioned within the hollow primary lumen, and wherein the channel catheter comprises a channel lumen;
a steerable navigation catheter, wherein the steerable navigation catheter is configured to fit within the channel lumen, and wherein the steerable navigation catheter comprises a steerable tip and position sensing element; and
an energy delivery device, wherein the energy delivery device is configured to fit within the channel lumen, and wherein the energy delivery device comprises, within the channel lumen, first, second and third conductors therein.

8. The kit of claim 7, wherein the primary catheter comprises a bronchoscope.

9. The kit of claim 7, wherein the energy delivery device is a microwave ablation device or a radiofrequency energy delivery device.

10. The kit of any of claims 7 to 9, further comprising a handle for manipulation of one or more of the primary catheter, the channel catheter, the steerable navigation catheter, and the energy delivery device.

11. The kit of any of claims 7 to 10, further comprising a processor configured to operate power delivery to the energy delivery device.

12. The kit of any of claims 7 to 11, wherein the energy delivery device is capable of delivering microwave energy through the channel catheter.

13. The kit of any of claims 7 to 12, wherein the energy delivery device comprises a coolant channel and wherein the system comprises a coolant source in fluid communication with the coolant channel.

14. A method of securing a medical device in a desired position, comprising
securing the apparatus (1) of any of claims 1 to 6 to an anchorable region,
securing a medical device with the medical device attachment region distal end (8), and
adjusting the apparatus such that the secured medical device is in a desired position, wherein the adjusting comprises adjusting the projection of the medical device attachment region (4) from the connection region (5).

15. The method of claim 14, wherein the medical device is an energy delivery device configured to ablate tissue.

## Patentansprüche

1. Eine Vorrichtung (1) zum Sichern eines medizinischen Geräts, beinhaltend:
einen länglichen Hauptkörper (2) mit einem proximalen Hauptkörperende (6) und einem distalen Hauptkörperende (7), wobei die Länge des Hauptkörpers größer als die Breite des Hauptkörpers ist,
einen Verankerungsbereich (3), der mit dem proximalen Hauptkörperende in Eingriff steht, wobei der Verankerungsbereich konfiguriert ist, um auf einem verankerbaren Bereich zu verriegeln, wobei beim Verriegeln mit einem verankerbaren Bereich der Verankerungsbereich und der längliche Hauptkörper in einer festen Position verriegelt sind,
einen Befestigungsbereich (4) für ein medizinisches Gerät mit einem distalen Ende (8) des Befestigungsbereichs für ein medizinisches Gerät und einem proximalen Ende (9) des Befestigungsbereichs für ein medizinisches Gerät,
wobei das proximale Ende des Befestigungsbereichs für ein medizinisches Gerät länglich ist, sodass die Länge des proximalen Endes des Befestigungsbereichs für ein medizinisches Gerät größer als die Breite des proximalen Endes des Befestigungsbereichs für ein medizinisches Gerät ist,
wobei das distale Ende des Befestigungsbereichs für ein medizinisches Gerät konfiguriert ist, um ein medizinisches Gerät zu sichern, und
einen Verbindungsbereich (5), der mit dem distalen Hauptkörperende und dem proximalen Ende des Befestigungsbereichs für ein medizinisches Gerät in Eingriff steht,
wobei der Verbindungsbereich einstellbar ist, sodass der Befestigungsbereich für ein medizinisches Gerät in jeder Richtung davon verriegelbar vorstehen kann, d. h. mit einem radialen Vorsprung von 0° bis 360° und einem Drehvorsprung von 0° bis 360° um eine Achse des Verbindungsbereichs.

2. Vorrichtung gemäß Anspruch 1, wobei der Hauptkörper eine Breite aufweist, die zwischen 0,005 Metern und 0,3 Metern beträgt.

3. Vorrichtung gemäß Anspruch 1 oder Anspruch 2, wobei der Verankerungsbereich einen komprimierbaren Bereich des Verankerungsbereichs und einen Kompressorbereich des Verankerungsbereichs aufweist, wobei der Kompressorbereich des Verankerungsbereichs konfiguriert ist, um einen Kompressionsbandbreite auf den komprimierbaren Bereich des Verankerungsbereichs auszuüben, sodass die Größe des komprimierbaren Bereichs des Verankerungsbereichs mit zunehmenden Kompressionsbeträgen abnimmt und mit abnehmenden Kompressionsbeträgen zunimmt, wobei der Betrag der ausgeübten Kompression verriegelbar ist.

4. Vorrichtung gemäß einem der vorhergehenden Ansprüche, wobei das distale Ende des Befestigungsbereichs für ein medizinisches Gerät mit einem torsionsbasierten Sicherungsmechanismus konfiguriert ist, wobei der torsionsbasierte Sicherungsmechanismus naturgemäß in einer geschlossenen Weise ruht, wobei der torsionsbasierte Sicherungsmechanismus konfiguriert ist, um für Zwecke des Unterbringens eines medizinischen Geräts geöffnet zu werden, wobei das Lösen des torsionsbasierten Sicherungsmechanismus aus einer geöffneten Weise naturgemäß zu einem torsionsbasierten Schließen des torsionsbasierten Sicherungsmechanismus führt.

5. Vorrichtung gemäß einem der vorhergehenden Ansprüche, wobei der Befestigungsbereich für ein medizinisches Gerät darauf eine oder mehrere Verlängerungen aufweist, die davon vorstehen, wobei die eine oder die mehreren Verlängerungen konfiguriert sind, um ein oder mehrere medizinische Geräte zu sichern.

6. Vorrichtung gemäß einem der vorhergehenden Ansprüche, wobei das proximale Ende des Befestigungsbereichs für ein medizinisches Gerät eine Breite aufweist, die zwischen 0,025 Metern und 0,3 Metern beträgt.

7. Ein Kit, beinhaltend:
eine Vorrichtung (1) gemäß einem der vorhergehenden Ansprüche; und
ein System, wobei das System Folgendes beinhaltet:
einen Primärkatheter, wobei der Primärkatheter ein hohles primäres Lumen beinhaltet;
einen Kanalkatheter, wobei der Kanalkatheter konzentrisch innerhalb des hohlen primären Lumens positioniert ist und wobei der Kanalkatheter ein Kanallumen beinhaltet;
einen steuerbaren Navigationskatheter, wobei der steuerbare Navigationskatheter konfiguriert ist, um in das Kanallumen zu passen, und wobei der steuerbare Navigationskatheter ein steuerbares Neigungs- und Positionserfassungselement beinhaltet; und
ein Energieabgabegerät, wobei das Energieabgabegerät konfiguriert ist, um in das Kanallumen zu passen, und wobei das Energieabgabegerät innerhalb des Kanallumens einen ersten, zweiten und dritten Leiter darin beinhaltet.

8. Kit gemäß Anspruch 7, wobei der Primärkatheter ein Bronchoskop beinhaltet.

9. Kit gemäß Anspruch 7, wobei das Energieabgabegerät ein Mikrowellenablationsgerät oder ein Hochfrequenzenergieabgabegerät ist.

10. Kit gemäß einem der Ansprüche 7 bis 9, ferner beinhaltend einen Griff zur Handhabung von einem oder mehreren von dem Primärkatheter, dem Kanalkatheter, dem steuerbaren Navigationskatheter und dem Energieabgabegerät.

11. Kit gemäß einem der Ansprüche 7 bis 10, ferner beinhaltend einen Prozessor, der konfiguriert ist, um die Leistungsabgabe an das Energieabgabegerät zu betreiben.

12. Kit gemäß einem der Ansprüche 7 bis 11, wobei das Energieabgabegerät in der Lage ist, Mikrowellenenergie durch den Kanalkatheter abzugeben.

13. Kit gemäß einem der Ansprüche 7 bis 12, wobei das Energieabgabegerät einen Kühlmittelkanal beinhaltet und wobei das System eine Kühlmittelquelle in Fluidkommunikation mit dem Kühlmittelkanal beinhaltet.

14. Ein Verfahren zum Sichern eines medizinischen Geräts in einer gewünschten Position, beinhaltend:
Sichern der Vorrichtung (1) gemäß einem der Ansprüche 1 bis 6 an einem verankerbaren Bereich,
Sichern eines medizinischen Geräts mit dem distalen Ende (8) des Befestigungsbereichs für ein medizinisches Gerät und
Einstellen der Vorrichtung, sodass sich das gesicherte medizinische Gerät in einer gewünschten Position befindet, wobei das Einstellen das Einstellen des Vorsprungs des Befestigungsbereichs (4) für ein medizinisches Gerät von dem Verbindungsbereich (5) beinhaltet.

15. Verfahren gemäß Anspruch 14, wobei das medizinische Gerät ein Energieabgabegerät ist, das konfiguriert ist, um Gewebe zu ablatieren.

## Revendications

1. Un appareil (1) destiné à fixer solidement un dispositif médical, comprenant un corps principal allongé (2) ayant une extrémité proximale de corps principal (6) et une extrémité distale de corps principal (7), la longueur du corps principal étant supérieure à la largeur du corps principal,
une région d'ancrage (3) en prise avec l'extrémité proximale de corps principal, la région d'ancrage étant configurée pour se verrouiller sur une région apte à être ancrée, la région d'ancrage et le corps principal allongé étant verrouillés dans une position fixe lors du verrouillage avec une région apte à être ancrée,
une région d'assujettissement de dispositif médical (4) ayant une extrémité distale de région d'assujettissement de dispositif médical (8) et une extrémité proximale de région d'assujettissement de dispositif médical (9),
dans lequel l'extrémité proximale de région d'assujettissement de dispositif médical est allongée de telle sorte que la longueur de l'extrémité proximale de région d'assujettissement de dispositif médical est supérieure à la largeur de l'extrémité proximale de région d'assujettissement de dispositif médical,
dans lequel l'extrémité distale de région d'assujettissement de dispositif médical est configurée pour fixer solidement un dispositif médical, et
une région de raccordement (5) venant en prise avec l'extrémité distale de corps principal et l'extrémité proximale de région d'assujettissement de dispositif médical, la région de raccordement étant réglable de telle sorte que la région d'assujettissement de dispositif médical peut faire saillie de manière verrouillable dans n'importe quelle direction à partir de celle-ci, c'est-à-dire avec une saillie radiale allant de 0° à 360° et une saillie de rotation allant de 0° à 360° autour d'un axe de la région de raccordement.

2. L'appareil de la revendication 1, dans lequel le corps principal a une largeur qui est comprise entre 0,005 mètre et 0,3 mètre.

3. L'appareil de la revendication 1 ou de la revendication 2, dans lequel la région d'ancrage a une région compressible de région d'ancrage et une région de compression de région d'ancrage, la région de compression de région d'ancrage étant configurée pour appliquer une plage de compression sur la région compressible de région d'ancrage de telle sorte que la taille de la région compressible de région d'ancrage diminue avec des quantités accrues de compression et augmente avec des quantités diminuées de compression, la quantité de compression appliquée étant verrouillable.

4. L'appareil de n'importe quelle revendication précédente, dans lequel l'extrémité distale de région d'assujettissement de dispositif médical est configurée avec un mécanisme de fixation solide basée sur la torsion, le mécanisme de fixation solide basée sur la torsion reposant naturellement d'une manière fermée, le mécanisme de fixation solide basée sur la torsion étant configuré pour être ouvert dans le but d'accueillir un dispositif médical, le relâchement du mécanisme de fixation solide basée sur la torsion d'une manière ouverte ayant naturellement pour résultat une fermeture basée sur la torsion du mécanisme de fixation solide basée sur la torsion.

5. L'appareil de n'importe quelle revendication précédente, dans lequel la région d'assujettissement de dispositif médical a sur celle-ci une ou plusieurs extensions faisant saillie à partir de celle-ci, les une ou plusieurs extensions étant configurées pour fixer solidement un ou plusieurs dispositifs médicaux.

6. L'appareil de n'importe quelle revendication précédente, dans lequel l'extrémité proximale de région d'assujettissement de dispositif médical a une largeur qui est comprise entre 0,025 mètre et 0,3 mètre.

7. Un kit comprenant :
un appareil (1) de n'importe quelle revendication précédente ; et
un système, le système comprenant :
un cathéter primaire, le cathéter primaire comprenant une lumière primaire creuse ;
un cathéter à canal, le cathéter à canal étant positionné de manière concentrique à l'intérieur de la lumière primaire creuse, et le cathéter à canal comprenant une lumière de canal ;
un cathéter de navigation orientable, le cathéter de navigation orientable étant configuré pour s'ajuster à l'intérieur de la lumière de canal, et le cathéter de navigation orientable comprenant un élément de détection d'inclinaison et de position orientable ; et
un dispositif d'apport d'énergie, le dispositif d'apport d'énergie étant configuré pour s'ajuster à l'intérieur de la lumière de canal, et le dispositif d'apport d'énergie comprenant, à l'intérieur de la lumière de canal, des premier, deuxième et troisième conducteurs dans celui-ci.

8. Le kit de la revendication 7, dans lequel le cathéter primaire comprend un bronchoscope.

9. Le kit de la revendication 7, dans lequel le dispositif d'apport d'énergie est un dispositif d'ablation par micro-ondes ou un dispositif d'apport d'énergie radiofréquence.

10. Le kit de n'importe lesquelles des revendications 7 à 9, comprenant en outre une poignée destinée à la manipulation d'un ou de plusieurs éléments parmi le cathéter primaire, le cathéter à canal, le cathéter de navigation orientable, et le dispositif d'apport d'énergie.

11. Le kit de n'importe lesquelles des revendications 7 à 10, comprenant en outre un processeur configuré pour faire fonctionner un apport d'alimentation au dispositif d'apport d'énergie.

12. Le kit de n'importe lesquelles des revendications 7 à 11, dans lequel le dispositif d'apport d'énergie est apte à apporter de l'énergie micro-ondes à travers le cathéter à canal.

13. Le kit de n'importe lesquelles des revendications 7 à 12, dans lequel le dispositif d'apport d'énergie comprend un canal de fluide de refroidissement et dans lequel le système comprend une source de fluide de refroidissement en communication fluidique avec le canal de fluide de refroidissement.

14. Un procédé consistant à fixer solidement un dispositif médical dans une position souhaitée, comprenant
la fixation solide de l'appareil (1) de n'importe lesquelles des revendications 1 à 6 à une région ancrable,
la fixation solide d'un dispositif médical avec l'extrémité distale de région d'assujettissement de dispositif médical (8), et
le réglage de l'appareil de telle sorte que le dispositif médical solidement fixé est dans une position souhaitée, le réglage comprenant le réglage de la saillie de la région d'assujettissement de dispositif médical (4) à partir de la région de raccordement (5).

15. Le procédé de la revendication 14, dans lequel le dispositif médical est un dispositif d'apport d'énergie configuré pour ablater un tissu.
